Europäisches Patentamt

European Patent Office

Office européen des brevets

(11) Publication number : **0 512 785 A1**

## (12) EUROPEAN PATENT APPLICATION

(21) Application number : **92304022.4**

(22) Date of filing : **05.05.92**

(51) Int. Cl.⁵ : **A61F 2/16**

(30) Priority : **04.05.91 EG 9105266**

(43) Date of publication of application :
**11.11.92 Bulletin 92/46**

(84) Designated Contracting States :
**DE FR GB SE**

(71) Applicant : **Daif, Mohamed Abd El Fattah
Hassan, Dr.
9 Zakzouk St. El Masakin
El Taawnia, El Zakazik (EG)**

(72) Inventor : **Daif, Mohamed Abd El Fattah
Hassan, Dr.
9 Zakzouk St. El Masakin
El Taawnia, El Zakazik (EG)**

(74) Representative : **Baillie, Iain Cameron et al
Ladas & Parry Altheimer Eck 2
W-8000 München 2 (DE)**

(54) **Intraocular lens implant.**

(57)    An intraocular lens implant has two portions with a releasable member, preferably a 9/0, 0.3 metric nylon suture, for tying the two portions together to reduce their combined size and to maintain the portions as a single unit for implantation into the eye. The implant may be provided in a tied or half tied condition.

There are also disclosed two lens designs one preferably having a 6mm diameter optical zone and three shoulders on the circumference of the optic at equal distances with each having a 0.3mm hole and 12.5mm fixed curve loop of 0.2mm transverse sectional diameter extending in an anticlockwise direction. The second lens preferably has a 6mm by-optical zone attached via three loops each being one 6th of a circle, to a fixed circle that comprisses three main arcs.

A single-way sterile narrow plastics I.V. cannula is further described having a tip which is bevelled obliquely and a tooth is located on each side of the bevel for engaging a sterile stainless steel wire in a loop form to thread suture ends through the cannula.

Fig. (2)

EP 0 512 785 A1

This invention relates to an intraocular lens implant.

In order to make intraocular lens implantation easier, the lens, while being introduced to the empty crystalline lens capsular bag, should be made smaller and all its parts should move as one unit. If the instrumentation, used during implantation, is reduced to a 22 G sterile plastic I.V. cannula and an IOL manipulation hook with or without IOL holding forceps and there is a continuous gentle irrigation of the anterior chamber and the capsular bag with B.S.S. through the cannula, extra advantages can be therefore achieved. All of the above mentioned advantages can be achieved if External Tie And Internal Release (E.T.A.I.R.) Method for IOL implantation is used.

According to one aspect of the present invention there is provided an intraocular lens implant having at least two portions, characterised in releasable means for tying the at least two portions together to reduce their combined size and to maintain the portions as a single unit for implantation into the eye.

According to another aspect of the invention there is provided a single-way I.V. cannula, characterised in that the cannula has a tip which is bevelled obliquely and a tooth located on each side of the bevel for engaging a sterile stainless steel wire in a loop form to thread suture ends through the cannula. The cannula preferably a single-way sterile narrow plastics I.V.cannula.

Embodiments of the invention will now be described by way of example with reference to the accompanying drawings in which:-

Fig. 1 is a known Pannu lens;

Fig. 2 is the lens of Fig. 1 together with a suture for tying the lens;

Fig. 3 illustrates an intraocular lens implant in a tied condition;

Fig. 4 illustrates a disposable I.V. cannula;

Fig. 5 illustrates the bevelling of one end of the cannula;

Figs 6, 6a, and 6b illustrate a bevelled end of the cannula;

Fig. 7 is a diagrammatic view of a thin sterile stainless steel loop to be passed through the lumen of the cannula;

Fig. 8 represents an eye with the cataract removed;

Fig. 9 illustrates insertion of the intraocular lens implant into the eye with the ends of the suture extending outwardly of the eye;

Fig. 10 illustrates the single unit lens located in position in the eye with the suture ends external of the eye;

Figs. 11 to 13 illustrate location of the suture ends through the wire loop, pulling the suture ends through the lumen of the cannula, and for holding the suture ends whilst the implant is rotated;

Fig. 14 illustrates location of the suture in cuts behind the teeth of the bevelled edges of the cannula to fix the cannula to the suture;

Fig. 15 illustrates the cutting of the suture;

Fig. 16 illustrates withdrawal of the suture through the lumen of the cannula;

Fig. 17 illustrates removal of the cannula and securing of the wound with a plurality of interrupted sutures;

Figs. A1-a and A1-b are respectively front and side views of an intraocular lens implant having a central

Figs. A2 and A3 illustrate methods of tying the lens with the suture;

Figs. B1-a and B1-b are front and side views of an implant respectively of an intraocular lens implant having an outer fixing ring;

Fig. B2 illustrates the lens after tying the implant;

Figs. B3, B4 and B5 each illustrate one third of an intraocular lens each of different sizes to meet the requirements of people of differing age groups.

Referring now to the drawings in more detail:

## THE E.T.A.I.R. METHOD

- The Pannu lens∗ (Fig. 1) is used for demonstrating the method as it is more or less suitable though there are some precautions.

- A nylon 9/0 suture∗∗ is used for tying the lens with way∗∗∗ shown in Fig. 2. When the suture is stretched and tied, the lens takes the shape shown in Fig. 3 which is generally oval with a transverse axis not more than 7 mm, and a long axis not more than 9 mm. The suture ends would be kept long after the knot.

∗ PANNU Style AP-55 B 14mm according to Allergan Medical Optics Product Catalog.
∗∗ Nylon 9/0-0.3 metric, according to Ethicon product catalog.
∗∗∗ Bruner, W.E. Stark, W.J. and Maumenee A.E.Modified adjustable slip-knot technique for typing nylon sutures, surgery of the lens, Rob & Smiths Surgery, Ophthalmic Surgery, Fourth Edition, Butterworths, P.150, 1984.

- A sterile 22 G, or narrower, disposable I.V. cannula (as shown inn Fig. 4) is prepared to be one of the instruments used after removal of themetal trocar. This is done by bevelling its end with a sharp knife blade as shown in Fig. 5 and Fig. 6. Then another cut is made as shown in Fig. 6-a so that each edge of the bevel would have a tooth and the final shape will be shown in Fig. 6-b.

- Also, a thin sterile stainless-steel loop (Fig.7) is prepared so that it can be passed easily through the lumen of the cannula.

## Steps of ETAIR Method

**1.** The cataract is removed by E.C.C. Ex. (or by Endo. or Inter. C.C.Ex.) Method (Fig. 8). Healon may or may not be injected to protect corneal endothelium and to facilitate insertion.

**2.** The tied lens is introduced to the inside of the eye in the empty lens capsular bag with a holding forceps so that its long axis is in the same direction of insertion (Fig. 9). The ends of the suture, behind the knot, are kept outside the eye.

**3.** As the tied lens is made smaller and moves as one unit, it is easy to control it and to implant it exactly in the empty capsular bag (Fig. 10).

**4.** The stainless-steel wire is passed across the lumen of the cannula so that its looped end is passed first until the looped end appears from the bevelled end of the cannula. Then, the suture ends are passed through the loop (Fig. 11).

**5.** The stainless-steel wire is withdrawn so that the loop pulls the suture ends threading them across the lumen of the cannula (Fig. 12).

**6.** The cannula is gently introduced to the inside of the eye guided by the suture ends which are pulled gently during the introduction of the cannula until its bevelled tip reaches the middle of the lens front surface so that the bevel of the cannula faces the cornea. Then, an IOL manipulation hook is introduced to the inside of the eye and the IOL is rotated from the axilla of the lower lens fixation loop in a clockwise direction till the suture passing between the holes of lens optic is made transverse (Fig. 13).

**7.** The IOL manipulation hook is removed and the ends of the suture are fixed so that they become tense without moving the lens. The cannula is then pushed to the inside gently so that the tension in the suture ends would neutralize the pushing force exerted by the cannula on the lens. Therefore, the lens would not move. At the same time, tension on the suture ends elevates the knot and the transverse suture at its sides to a slightly higher level than that of the lens surface so that the tip of the beveled end of the cannula can pass between them. Further pushing of the cannula will elevate the sutures gradually along the edges of the bevelled end till the transverse suture falls in the cuts behind the teeth of the bevelled edges to fix the cannula to the suture and accordingly to the lens (Fig. 14).

**8.** Closed tips of a long-bladed microvannas scissors are introduced to the inside of the eye beside the cannula till they approach the transverse suture. The blades are slightly opened so that they catch the suture inbetween with the blade beside the cannula passing below the suture. The blades are then closed to cut the suture so that the lens fixation loops are released taking their positions inside the capsular bag and performing their function, (Fig. 15). Then the scissors are removed.

**9.** After cutting the tying suture and releasing the lens fixation loops, the suture is withdrawn through the lumen of the cannula by pulling its external ends (Fig. 16).

**10.** After making sure that everything is alright and the lens is in Place, the cannula is withdrawn and the wound is secured with a suitable number of interrupted sutures (Fig. 17).

**xx.** It is possible to rotate the IOL inside the lens capsular bag before threading the suture ends through the cannula and before introducing the cannula to the inside of the eye.

**xx.** It is possible to thread the suture ends through the cannula and to push the cannula to fixate the cannula to the suture and accordingly to the lens before introducing the lens to the inside of the eye. In this case, the cannula is used as an introducer and a guide to the IOL during its implantation. Also, the lumen of the cannula is used to irrigate the eye gently with B.S.S. during implantation.

## Advantages of External Tie And Internal Release Method (ETAIR):

**1.** Simplicity, as it needs neither special skills nor long experience.

**2.** Sure in the bag implantation of the IOL as in the tied form it becomes smaller and moves as one unit so that it can be controlled easily.

**3.** The tied lens can be easily moved inside the eye or even extracted as long as the suture is not cut.

**4.** It does not require sofisticated or expensive instruments. So, it will not cost the surgeon any extra-expenses.

5. If the cannula is fixed to the suture and the IOL, before introducing the IOL, this will add two extra-advantages:

a) The cannula will act as an introducer and a guide to the IOL which will, together with an IOL manipulation hook, allow better IOL control and also will make IOL holding forceps unnecessary.

b) The cannula will allow gentle irrigation of the A.C. with B.S.S. during implantation.

## Precautions about the Compatibility of Pannu Lens Design with ETAIR Method :

**1.** As it has only two fixation loops, the sutures, passing between the two holes of the optic, cross the anterior lens surface at its centre. So, the central part is liable to scratch during cutting the suture.

**2.** As its fixation loops are at the same plane with the optic, tying the loops behind the optic may lead to their moulding so that they would keep some posterior tilt after releasing them. This may lead, after implantation, to the wrinkling of the posterior lens capsule behind the IOL and also to the anterior displacement of the IOL optic towards the pupil followed by the anterior vitreous face behind the posterior lens capsule.

**3.** Releasing the fixation loops, after tying them, may lead to their moulding so that the distance between the two farthest points on lens loops would not be 14 mm. However, as the diameter of the empty lens capsule is about 10 mm., there are 4 mm. reserve.

## Special Precautions :

**1.** Using the I.V. plastic cannula inside the eye is considered to be a new use. But as it is manufactured to stay in a vein up to 48 hours and as it will not be used inside the eye except for few minutes, no problems will be expected from this use.

**2.** Using the nylon 9/0 suture inside the empty capsular bag is also considered to be a new use. But as this suture is inert and also as it is monofilament, no problems will be expected from this use.

## DESIGN (1), AN APPLICATION OF ETAIR METHOD DAIF-I-POSTERIOR CHAMBER-INTRAOCULAR LENS (DAEF—I-PC—IOL)

## Rationale :

The External Tie And Internal Release (ETAIR) Method facilitates IOL implantation and makes it very simple and easy. However, there is no single IOL design, at present time, that can be considered to be fully suitable to and compatible with this method. So, there is a demand for having a new lens design that suits the ETAIR method. DAIF-I-PC-IOL is designed specifically to satisfy this demand.

## DAIF-I-PC-IOL

## Description (Fig. A 1-a & A 1-b):

- It is made up of one piece of Perspex∗ CQ PMMA or better of PMMA with C.M. Technology∗∗, each with U.V. blockers.
- It has an optical part and fixation parts.
- The optical part is a 6mm. diameter biconvex lens that is more convex posteriorly (2:1). The magnitude of curvature is in accordance to its power.
- The fixation parts are three shoulders fused to the optical part (optic), each with a maximal height of 0.5 mm. They lie at equal distances on the circumference of the optic.
- Each shoulder has an 0.3 mm. diameter hole making its inner edge 3mm. away from the optic centre, i.e., the distance equals the optic radius.
- From each shoulder, an 0.2 cross-sectional diameter fixation loop emerges in an anticlockwise direction. Each loop is a modified-C-loop of compound curve that ends with a diamond shaped piece of 1.4 mm. long axis and an 0.7 mm. short axis. Its long axis makes an angle of 150° with the loop at the meeting point. This diamond shaped piece has a central 0.3 mm. hole. The angles of this piece are made smooth, not sharp. The length of each loop from its exit to the end of the diamond shaped piece is 12.5 mm. and equals 2/3 the circumference of the optic. The three fixation loops at their farthest points from the optical axis touch

∗ Perspex CQ is a trademark of ICI, Ltd. - USA.
∗∗ Compression Moulding Technology of Kabi-Pharmacia Co. - Sweden.

a virtual circle of 14 mm. diameter around the optical axis, i.e., total diameter = 14 mm.

- The fixation loops are tilted anteriorly so that the end of each touches a virtual line that makes 10° with the optical plane at its edge.

- So, the lens has an optic, three shoulders and three fixation loops that make three axellae with the optic. The lens has 6 holes.

- The central part of the anterior surface of the optic can be made more convex with an additional power of 4 dioptres and with a radius of 1 mm. around the optical axis in order to make the lens bifocal.

- Smaller lenses can be manufatured with optical diameters of 5.5 mm. or 5 mm. The length of fixation loops and total diameters are accordingly reduced. This makes implantation more easier, but it may increase the spherical aberration and edge glare as the edge of optic will become nearer to the pupillary edge after implantation.

## Method of Implantation:

DAIF-I-PC-IOL is designed specifically to be suitable for and compatible with intraocular implantation according to External Tie And Internal Release (ETAIR) Method.

## Tying the lens (Fig. A 2 & fig A 3):

- The lens is tied using a single uninterrupted suture that tie the diamond shaped end of each loop, behind the optic, to the hole of the next second shoulder following its own in the direction of the emergence of the loop from its shoulder. When two successive loops cross each other behind the optic, the one that has nearer exit is the nearest to the optic (Fig. A 2).

- After tying, the lens takes the shape shown in Fig. (A 2) making each loop, in its farthest point, between 0.5 mm. and 1 mm. from the circumference of the optic (0.5 mm. is the height of each shoulder). So, the lens takes a rounded shape with three projections on equal distances. Therefore, the farthest distance between any two points in it will not exceed 7 mm. This gives the tied lens a great advantage during implantation into the empty capsular bag.

- The lens is tied with a nylon 9/0 suture, utilizing its holes the way shown in Fig. (A 3).

- The suture, on the anterior lens surface, takes the shape of an equidistant triangle and the knot is located at the middle of its base. From the knot, the two long suture ends exit. This base, at its midpoint, is located at half distance between the lens centre and edge.

- The lens is manufactured and distributed in the tied form as the tying process is too difficult to be done by the surgeon at surgery.

## Advantages of DAIF-I-PC-IOL:

**1.** It suits the ETAIR Method. So, it gets all its advantages

**2.** The farthest distance, between any two points in its tied form, does not exceed 7 mm. Its small size facilitates its control to be implanted for sure in the bag.

**3.** It is suitable for implantation after both E.C.C. Ex. or Inter. or Endo. C.C. Ex (Envelope Technique).

**4.** It is suitable for implantation inside the empty capsular bag as well as the ciliary sulcus fixation if the capsule is torn.

**5.** The anterior suture that carries the knot passes, at its middle, nearly at half distance between lens centre and edge. This makes the process of cutting the suture to be done away from the lens centre.

**6.** The suture behind the lens is minimum. So, its friction with the posterior lens capsule is minimal.

**7.** The optical part is free of holes.

**8.** The optical part is 6 mm. in diameter hence it decreases edge glare.

**9.** The fixation loops, when released while moving to reach their final position, partially scrape the posterior capsule. Also, their backward - forward movement assures the in the bag position of the lens.

**10.** Three fixation loops, instead of two, make the lens more stable inside the eye after implantation. They also increase the tension directions on the posterior lens capsule. So, it would become more homogenous. Also they markedly decrease the chance for decentralization, thus, making the lens suitable for work as a bifocal lens.

## Precaution:

After tying the lens loops for a long period, they may mould so that the loops might become more curved and less anteriorly tilted. This can be overcome by :

**a)** Decreasing loop curvature and increasing its anterior tilt when manufacturing it. Thus, on release, the result will be satisfactory. The amount of this improvement could be adjusted according to laboratory experimentations.

**b)** Tying the lens just before surgery by the surgeon. This needs a simple and cheap instrument that can be used by the surgeon. Its design can be easily made.

## DESIGN (2), AN APPLICATION OF ETAIR METHOD DAIF II POSTERIOR CHAMBER-INTRAOCULAR LENS (DAIF-II-PC-IOL)

### Rationale:

External Tie And Internal Release (ETAIR) Method facilitates IOL implantation and makes it very simple and easy. However, there is no single IOL design, at present time, that can be considered to be fully suitable for and compatible with this method. So, there is a demand for having a new lens design that suits the ETAIR Method. If this design is made in different sizes to accommodate different age groups, extra advantages will be achieved. DAIF-II-PC-IOL is designed specifically to satisfy this demand.

### DAIF-II-PC-IOL:

### Description:

- It is made up of one piece of Perspex∗ CQ PMMA or better of PMMA with C.M. Technology ∗∗.
- The optical part is a biconvex lens so that the curvature of the posterior surface is nearly twice as big as the anterior one (2:1). The optical part is 6 mm. in diameter.
- The fixation parts are three curved loops that take attachment from the anterior aspect of the circumference of the optic at equal distances from each other. Each loop is 1/6 circle and has a cross-sectional diameter of 0.2 mm. The other end of each loop is attached to the outer fixation ring (Fig. B 1-a & B 1-b).
- The outer fixation ring is an incomplete circle with an inner radius of 4.7 mm. and an outer radius of 4.9 mm., i.e., its total diameter is 9.8 mm., and its cross-sectional diameter is 0.2 mm. It is composed of 3 main arcs. Each adjacent two arcs are attached to each other by a linking half circle that has an inner radius of 0.6 mm. and an outer radius of 0.8 mm.
and has a cross-sectional diameter of 0.2 mm., too. The attachments of fixation arcs to the linking half circles are made by smooth gradual curves and not sharp angles (Fig. B 1-a).
- Each fixation arc is attached from its centre to the curved loop and to the edge of the optic (Fig. B 1-a & B 1-b).
- Each fixation arc is enforced at its centre with a crescentic part fused to it from inside. The anteroposterior thickness of this crescentic part is 0.2 mm. Its inner edge is 0.3 mm. distant from the opposite outer edge of the fixation arc at the farthest points. The inner radius of the crescentic part is one and half times (3/2) as large as the inner radius of the fixation arc (Fig. B 1-a).
- The optic has two side devices to facilitate tying. Each is 0.3 mm. away from the nearest curved loop and in the direction of the third loop. Each device is quarter a disc with a radius of 0.4 mm. and an antero-posterior thickness of 0.2 mm. (Fig. B 1-a, B 1-b and Fig. B 2).
- The outer fixation ring is in a plane anterior to that of the optic so that the virtual line that joins the lens edge and fixation circle edge makes an angle of 10° with the lens plane (Fig.B 1-b).
- The lens is to be implanted in the eye utilizing the ETAIR (,External Tie and Internal Release) Method after Inter. or Endo. C.C.Ex.
- Fig. (B 2) shows the shape of the lens after tying it. It shows three projections protruding from the optic. Each projection at its farthest point is about 4 mm. from the centre of the optic. The rest of the fixation ring is tied behind the optic. Thus, the farthest distance between any two points in the tied lens is about 8 mm.
- A nylon 9/0 suture is used to tie the Lens in the way shown in Fig. (B 2).
- The lens is manufactured and distributed in the tied form.
- The lens is manufactured in different sizes to suit different age groups. The difference is in the total diameter of the fixation ring and accordingly in the curved loops (although each remains 1/6 circle) that become shorter. The same happens to the supporting crescents. However, the linking half circles between

∗ Perspex CQ is a Trademark of ICI, Ltd. USA.
∗∗Compession Moulding Technology of Kabi-Pharmacia Co. -Sweden.

fixation arcs retain their dimensions. The different size are:

**1)** A lens with the total diameter of 9.8 mm. that suits patients of 40 years of age or more (Fig. B 1-a, B 1-b, Fig B 2 and Fig. B 3) (Fig. B 3 shows 1/3 of the lens).

**2)** A lens with the total diameter of 9.2 mm. that suits patients of 10 years of age to less than 40 years. (Fig. B 4 that shows 1/3 of the lens).

**3)** A lens with the total diameter of 8.6mm. that suits patients of 1 year of age to less than 10 years (Fig. B 5 that shows 1/3 of the lens).

Table (1) shows the reference used to judge these dimensions.

- The curvature of the anterior central part of the optic can be increased in a diameter of 2 mm. and with the additional power of 4 dioptres to make the lens optic bifocal (Fig. B 3, B 4 & B 5). Hence, it would become suitable for near and far vision.

- The lenses that have fixation rings of the total diameter of 9.8 mm. when manufacturing bifocal types, can be made with an optical diameter of 6.4 mm. instead of 6 mm. in order to increase the part responsible for far vision (Fig. B 3).

## Advantages of DAIF-II-PC-IOL

**1.** It is suitable for implantation utilizing ETAIR Method. So, it utilizes all the advantages of this method.

**2.** After tying, the lens becomes smaller and more stable so it can be easily controlled to the inside of the empty capsular bag.

**3.** The front suture that carries the knot passes at its centre at nearly half distance between the centre of the optic and its edge. So, its cutting to release the fixation ring would be done away from the optical centre.

**4.** The optical zone is free of holes as their presence would decrease the optical effeciency of the lens.

**5.** The optical zone is 6 mm. in diameter so that the edge glare would be decreased.

**6.** The lens is suitable for implantation only in the undamaged empty capsular bag after Endo. or Inter. C.C.Ex. Its position inside the capsular bag gives it a stable position after the operation.

**7.** During its release, the fixation ring, while moving to its final position from backward to forward, partially scrapes the posterior capsule. This also assures in the bag position.

**8.** The presence of a fixation ring makes the lens very stable inside the capsular bag. It also prevents the anterior and equatorial epithelium from growing to the back of the optic.

**9.** The attachments of the optic to the fixation ring by three curved loops, instead of two, makes it balanced and stable at the centre of the ring. This is a great advantage that makes it suitable for manufacturing bifocal types as the greatest problem that limits the use of bifocal IOLs is decentralization.

**10.** The availability of the lens in different fixation ring diameters makes it suitable for different age groups. This, in addition to its central stability, may make its bifocal types to be the hope in treating soft cataracts in young ages that may, especially unilateral cases of soft cataract, lead to amblyopia which may be intractable.

**11.** The convexity of the posterior optical surface, in addition to the presence of the fixation ring in a plane anterior to that of the optic, causes the posterior capsule to be stretched behind the optic so that the chance for the posterior capsule to become wrinkled or opacified would be markedly minimized. Also, because this stretching on the posterior capsule is balanced and in all directions, not only in two opposite directions as all bilooped lenses, the maddox rod effect that can affect image clarity will be prevented.

**12.** Having a binconvex optic with more curved posterior surface makes the lens resemble the natural crystalline lens of the eye. So, the image formed by this IOL will resemble that of the natural lens.

**13.** The presence of multiple points of tension in the fixation parts, after tying (in the curved loops, fixation arcs, linking half circles and points of attachment of fixation arcs and linking half circles, will make the fixation parts more flexible and more resistant to moulding.

## Difficulty :

Because of the convexity of the posterior optical surface, using Nd-YAG laser, if the posterior capsule becomes opacified, to open the posterior capsule will need skilled hands to avoid damaging the optic.

| Ag* | Front Diameter of the * Human lens in mm, | Suggested Diameter of DAIF-II-PC-I.O.L. |
|---|---|---|
| 1-3 months | 6.31 | - |
| 3-11 months | 7.46 | |
| 1-10 years | 8.06 | 8.6 mm |
| 10-20 years | 8.47 | |
| 20-30 years | 8.67 | 9.2 mm |
| 30-40 years | 8.97 | |
| 40-50 years | 9.09 | |
| 50-60 years | 9.44 | |
| 60-70 years | 9.49 | 9.8 mm |
| 70-80 years | 9.64 | |
| 80-90 years | 9.62 | |

*Table (1)*

\* R. Weekers, Y. Delmarcello, J. Luyckx- Bacus and J. Collignon. Morphological changes of the lens with age and cataract, A Ciba Foundation Symposium (19). The Human lens in Relation to Cataract. Elsevier. Excerpta Medica. North Holllmd. Associated Scientific Publishers. Amesterdam. London. New York, Table (1), p. 27, 1973.

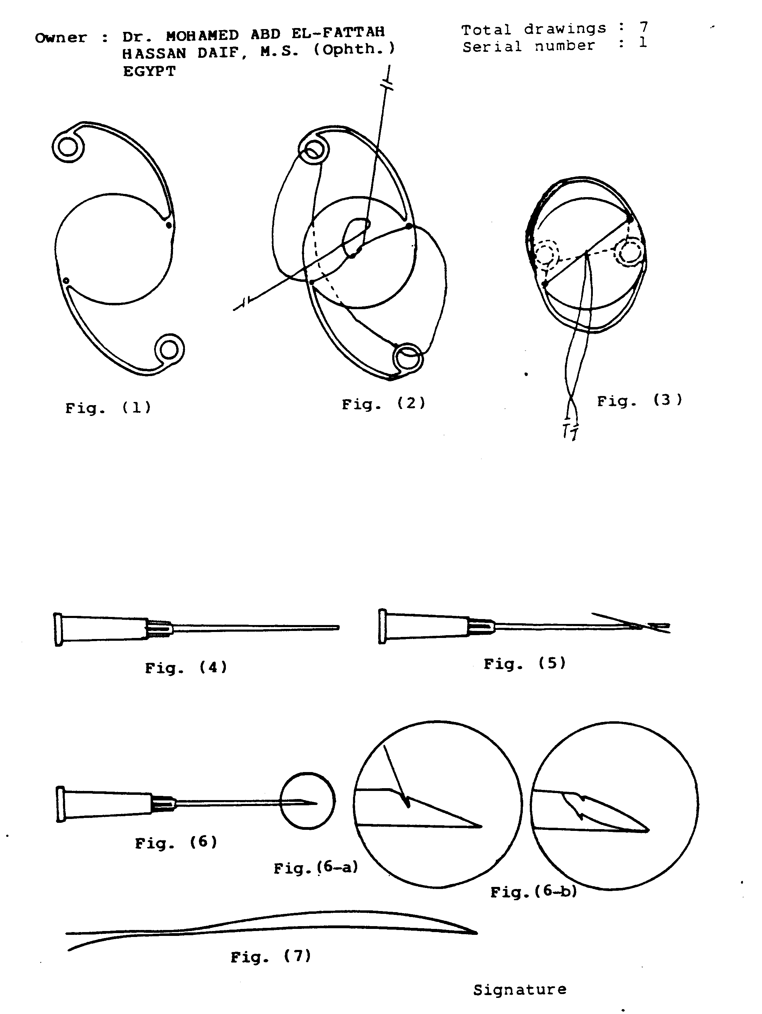

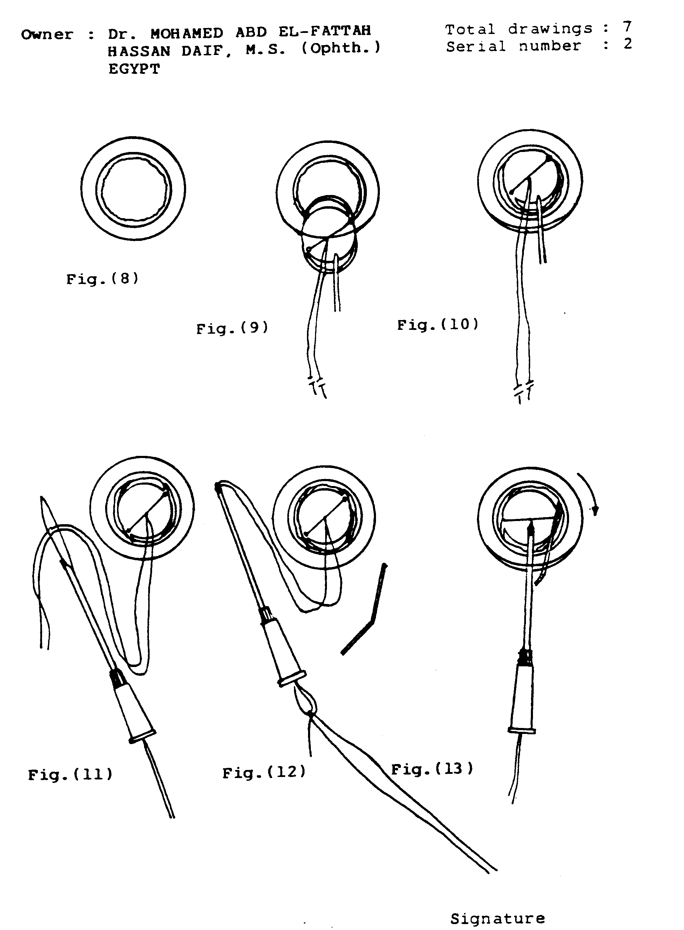

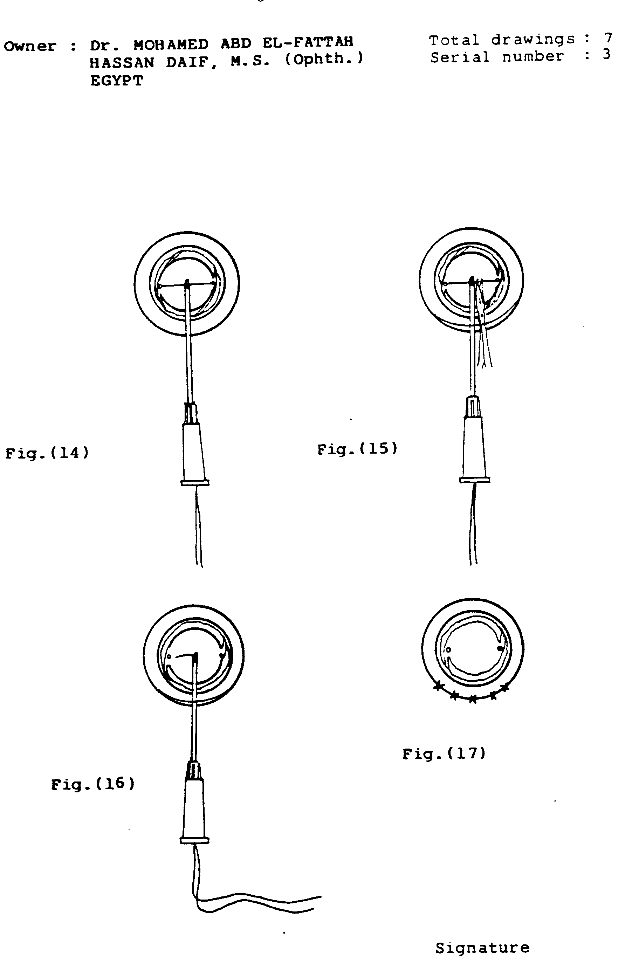

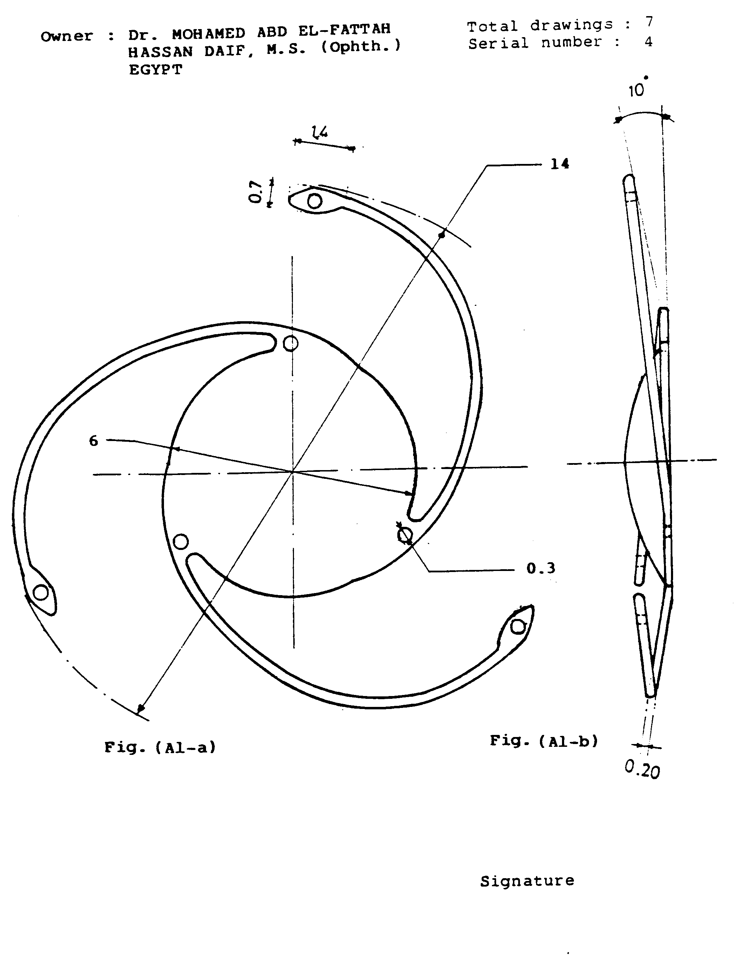

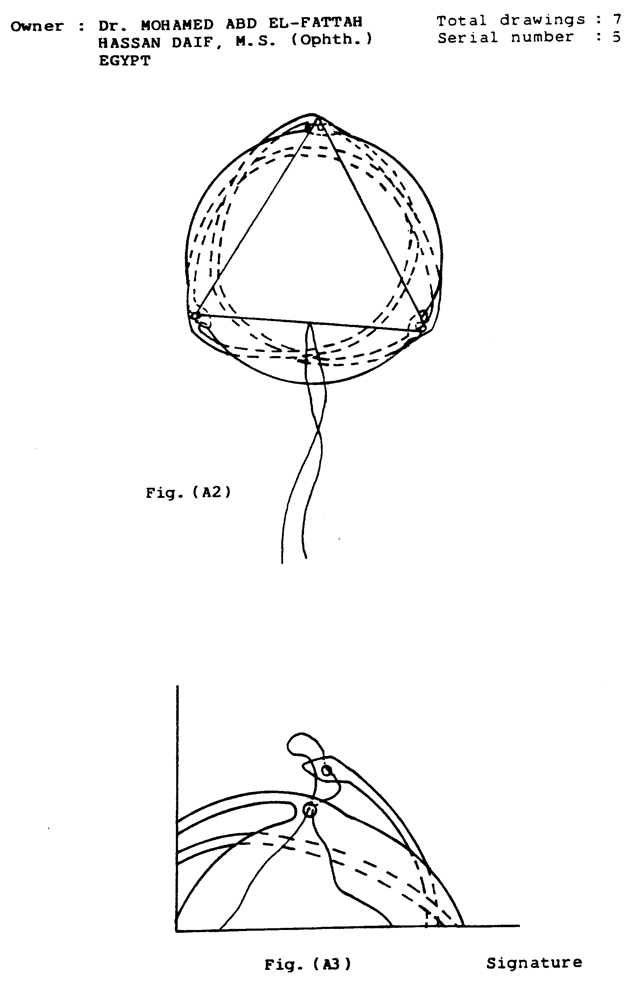

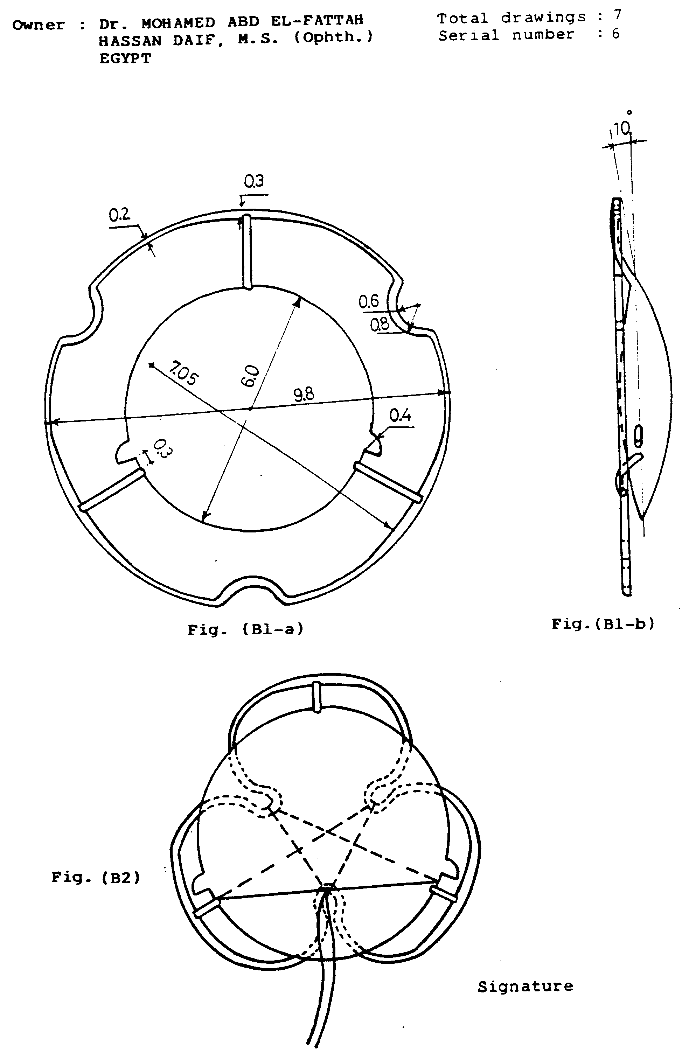

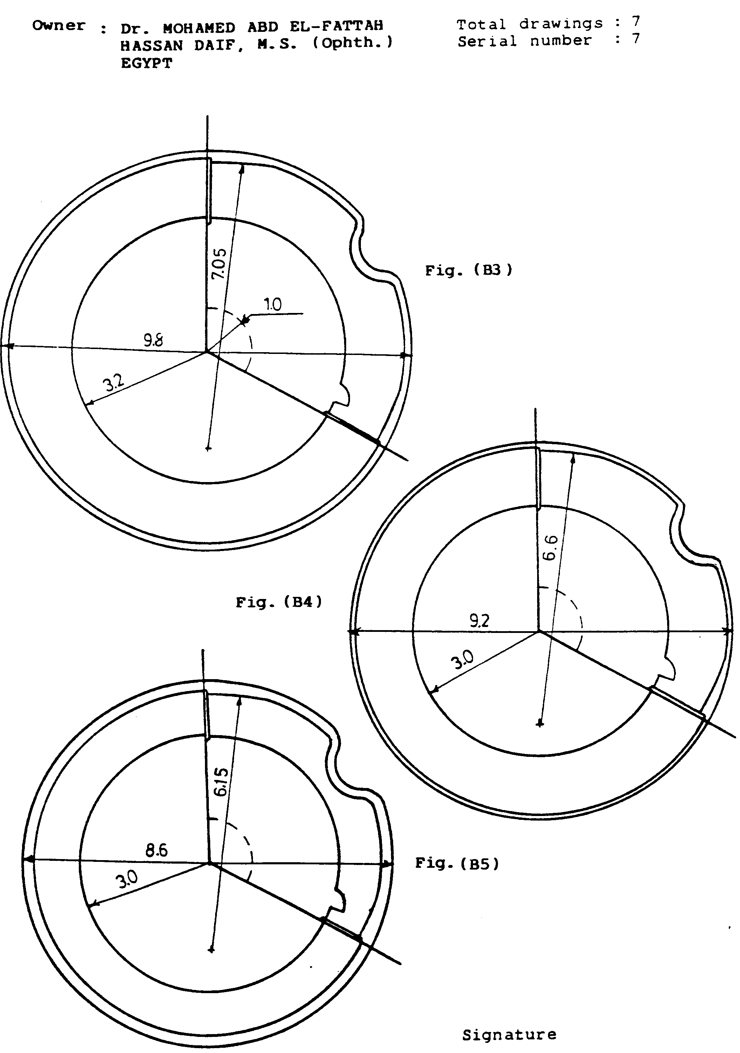

## Claims

1. An intraocular lens implant having at least two portions, characterised in releasable means for tying the at least two portions together to reduce their combined size and to maintain the portions as a single unit for implantation into the eye.

2. An implant as claimed in claim 1, characterised in that the releasable means is an uninterrupted thin sterile inert suture.

3. An implant as claimed in any one of the preceding claims, characterised in that the lens is provided in a tied form.

4. An implant as claimed in claim 5, characterised in that the tied form is a half tied form.

5. An intraocular lens, characterised in three curved fixation loops arranged to take exits from three shoulders located at equal distance on the circumference of the lens optic, each loop from its exit to its end having a length equal to two thirds of the length of the optic circumference, two holes located in each loop with one at its base in the shoulder and the other at its top in a diamond shaped member having an elongate axis and arranged to define an angle of 150° with the tangent of the axis of the fixation loop at their meeting point and the inner side, the holes having connections to the outside of the lens to facilitate tying of the lens and trapping of the suture inside the holes after tying it.

6. An intraocular lens, characterised in that each of three curved fixation loops defines an area equal to one 6th of a circle and takes exit from the anterior aspect of the lens optic circumference linking it to the centres of the main fixation arcs that define an outer fixation ring, the main fixation arcs being three arcs fused to each other from inside a supporting crescent part with an inner radius that equals one and half times that of the main fixation arc and at its furthest point from an opposite side of the point on the outer edge of the fixation arc by a distance equal to one and half times the diameter of the fixation arc without support, each adjacent two arcs being attachable to each other by a linking half circle that has an inner radius of 0. 6 mm an outer radius that exceeds the inner by a length equal to the cross-sectional diameter of the fixation

8

arc, the attachment of the fixation arcs to the linking half circles being smooth gradual curves, and in that the lens optic has two devices to facilitate tying, each being a quarter of a disc with a radius equal to twice the cross-sectional diameter of the fixation arc, and in that each device is spaced from the nearest curved loop in the direction of the third curved loop by a distance equal to one and a half times the cross-sectional diameter of the fixation arc.

7. A single-way I.V. cannula, characterised in that the cannula has a tip which is bevelled obliquely and a tooth located on each side of the bevel for engaging a sterile stainless steel wire in a loop form to thread suture ends through the cannula.

8. The cannula as claimed in claim 7, characterised in an intraocural lens manipulation hook for ensuring the suture on the anterior lens surface that carries the knot transverse when the implant is inserted into an empty crystalline lens capsular bag inside the eye after E.C.C.Ex. is left with the rest of the suture ends outside the eye, the prepared metal loop being usable to thread the suture ends through the prepared cannula whereupon the cannula is pushed to the inside of the eye guided by the suture ends and directed to the anterior lens surface, and in a pair of long bladed microvannas scissors the tips of which are introduceable into the eye to cut the transverse suture to set free the lens loops so that the said lens loops take their final positions prior to position of the wound.

9. The cannula as claimed in claims 7 or 8, characterised in that the cannula has a lumen so that when the cannula is tied to the implant prior to introduction into the empty crystalline lens capsula bag smooth gradual curves, and in that the lens optic has two devices to facilitate tying, each being a quarter of a disc with a radius equal to twice the cross-sectional diameter of the fixation arc, and in that each device is spaced from the nearest curved loop in the direction of the third curved loop by a distance equal to one and a half times the cross-sectional diameter of the fixation arc.

Owner : Dr. MOHAMED ABD EL-FATTAH
HASSAN DAIF, M.S. (Ophth.)
EGYPT

Total drawings : 7
Serial number : 1

Fig. (1)

Fig. (2)

Fig. (3)

Fig. (4)

Fig. (5)

Fig. (6)

Fig.(6-a)

Fig.(6-b)

Fig. (7)

Signature

10

Owner : Dr. MOHAMED ABD EL-FATTAH        Total drawings : 7
        HASSAN DAIF, M.S. (Ophth.)        Serial number : 2
        EGYPT

Fig.(8)

Fig.(9)

Fig.(10)

Fig.(11)

Fig.(12)

Fig.(13)

Signature

11

Owner : Dr. MOHAMED ABD EL-FATTAH
HASSAN DAIF, M.S. (Ophth.)
EGYPT

Total drawings : 7
Serial number : 3

Fig.(14)

Fig.(15)

Fig.(16)

Fig.(17)

Signature

Owner : Dr. MOHAMED ABD EL-FATTAH
HASSAN DAIF, M.S. (Ophth.)
EGYPT

Total drawings : 7
Serial number :   4

Fig. (Al-a)

Fig. (Al-b)

Signature

13

Owner : Dr. MOHAMED ABD EL-FATTAH          Total drawings : 7
         HASSAN DAIF, M.S. (Ophth.)          Serial number  : 5
         EGYPT

Fig. (A2)

Fig. (A3)                              Signature

Owner : Dr. MOHAMED ABD EL-FATTAH
HASSAN DAIF, M.S. (Ophth.)
EGYPT

Total drawings : 7
Serial number  : 6

Fig. (B1-a)

Fig.(B1-b)

Fig. (B2)

Signature

15

Owner : Dr. MOHAMED ABD EL-FATTAH
HASSAN DAIF, M.S. (Ophth.)
EGYPT

Total drawings : 7
Serial number  : 7

Fig. (B3)

Fig. (B4)

Fig. (B5)

Signature

European Patent Office

**EUROPEAN SEARCH REPORT**

Application Number

EP 92 30 4022

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 778 463 (J. HETLAND) | 1-4 | A61F2/16 |
| Y | * column 3, line 51 - line 68 *<br>* column 4, line 32 - line 35; figures * | 5 | |
| | --- | | |
| X | GB-A-2 167 306 (A.R. RICHARD) | 1,3 | |
| Y | * figures 7,8 * | 5 | |
| | --- | | |
| X | DE-A-3 722 910 (ADATOMED) | 1-4 | |
| Y | * column 4, line 30 - line 50 *<br>* column 6, line 62 - line 66; figures * | 6 | |
| | --- | | |
| Y | FR-A-2 530 457 (T.R. MAZZOCCO)<br>* page 14, line 11 - line 20; figure 4 * | 6 | |
| | --- | | |
| X | US-A-4 586 930 (C.D. KELMAN) | 1-4 | |
| A | * column 3, line 1 - line 46; figures 1,2 * | 6 | |
| | --- | | |
| A | US-A-4 624 670 (C.H. BECHERT,II)<br>* column 5, line 54 - column 6, line 14; figures 2,5 * | 6 | |
| | --- | | TECHNICAL FIELDS SEARCHED (Int. Cl.5) |
| X | US-A-4 699 140 (M.J. HOLMES ET AL.)<br>* abstract *<br>* column 9, line 35 - line 39; figures 4,10 * | 7 | A61F<br>A61B |
| | --- | | |
| A | US-A-4 346 713 (O. MALMIN)<br>* figures 12-16 * | 7 | |
| | --- | | |
| A | US-A-4 530 117 (C.D. KELMAN)<br>* abstract; figures * | 8 | |
| | ----- | | |

The present search report has been drawn up for all claims

| Place of search | Date of completion of the search | Examiner |
|---|---|---|
| THE HAGUE | 19 AUGUST 1992 | WOLF C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................
& : member of the same patent family, corresponding document

EPO FORM 1503 03.82 (P0401)